# EUROPEAN PATENT APPLICATION

(11) **EP 1 306 439 A1**
(43) Date of publication of application: **02.05.2003**
(21) Application number: 01124372.2
(22) Date of filing: 24.10.2001
(51) Int. Cl.: C12N 15/80, C12P 21/02, C12N 9/26, C07K 14/435

(54) **Protein production with non-metabolizable expression indicator**

(71) Applicant: Gesellschaft für Biotechnologische Forschung mbH (GBF), D-38124 Braunschweig (DE)
(72) Inventor: Ganzlin, Markus, Dr., 38124 Braunschweig (DE); Rinas, Ursula, Dr., 38124 Braunschweig (DE)
(74) Representative: Boeters, Hans Dietrich, Dr.

(57) **Abstract**

The invention concerns a process for the production of a protein by means of a microorganism, wherein
(i) a microorganism is used which is able to express a homologue or heterologue protein under the control of a promoter which is a glucoamylase-synthesis controlling promoter,
(ii) the expression is carried out in the presence of a non-metabolizable expression inductor and
(iii) the expressed protein is obtained.

## Description

The invention starts from the art of gene expression for production of proteins, wherein inductors of the expression are consumed.

According to one embodiment the invention is concerned with a process for the production of a protein by means of a microorganism, wherein
(i) a microorganism is used which is able to express a homologue or heterologue protein under the control of a promoter which is a glucoamylase-synthesis controlling promoter,
(ii) the expression is carried out in the presence of a non-metabolizable expression inductor and
(iii) the expressed protein is obtained.

A special embodiment of the invention concerns a process, wherein
(i) a promoter of or from a strain of Aspergillus, Saccharomyces, Candida or Lactobacillus or
(ii) a promoter is used which is encoded by a DNA, the complementary strand of which is hybridizable with the DNA encoding the promoter according to (i) under stringent conditions and which has a homology with said promoter according to (i) of at least 70 %, 80 %, 90 % or 95 %.

Thus, as promoter according to (i) a promoter of or from a strain of Aspergillus niger or Aspergillus oryzae can be used.

Thus, the glaA promoter of or from A. niger can be used.

Further, a special embodiment of the invention concerns a process, wherein the microorganism which is used for the expression of the protein is selected from the group of Aspergillus strains, Saccharomyces strains, Candida strains and Lactobacillus strains.

Thus, the Aspergillus strain can be selected from the group of A. niger and A. oryzae strains.

Further, a special embodiment of the invention concerns a process, wherein an A. niger strain according to EURO FUNG BIO4CT 960 535 is used.

According to the invention the promoter can be that of or from a strain which belongs to the same genus, species or strain of the microorganism which is used for the expression of the protein.

Further, a special emboidment of the invention concerns a process, wherein as non-metabolizable expression inductor an analogue of a starch hydrolysation product is used
(i) which has been subjected to a test for induction of the intended protein expression, found as inducing expression and as being not subject to a metabolization, and
(ii) which has been selected.

Further, a special embodiment of the invention concerns a process, wherein as non-metabolizable expression inductor an analogue of a starch hydrolysation product is used, wherein at least one oxygen atom has been exchanged by a sulfur atom.

Thus, as analogue of a starch hydrolysation product glucose or maltose can be used wherein at least one oxygen atom has been exchanged by a sulfur atom.

Thus, as non-metabolizable expression inductor 5-thio-glycose can be used.

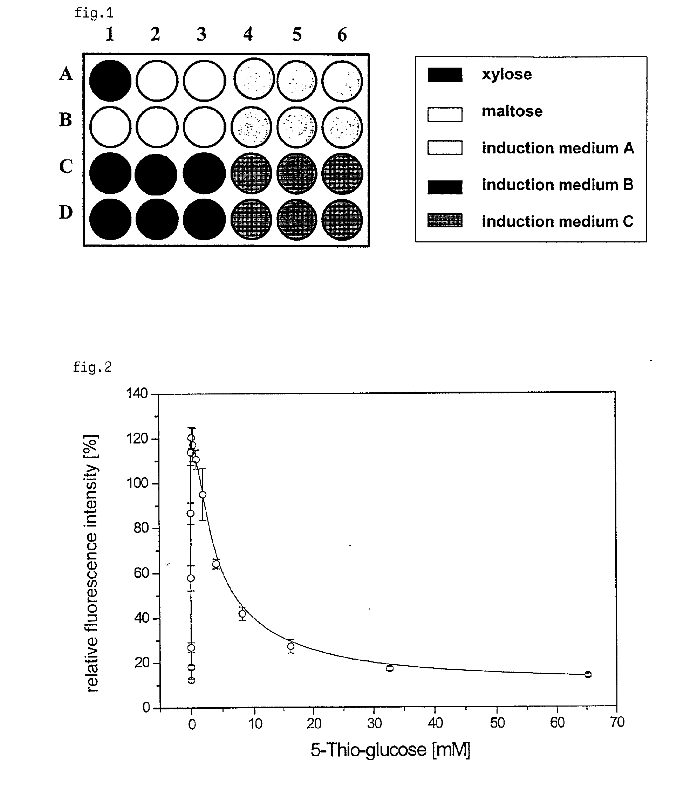

**Figure 1:** Arrangement of the media in the multiwell plates. The mycelium from the maltose positive control showed intensive fluorescence (RFI = 100%), whereas only low fluorescence intensity (RFI < 15%) was measured for the xylose negative control. An only minor deviation of the pH values (< pH 0.5) was detected after the incubation in different media.

**Figure 2:** Relative fluorescence intensity, determined by means of the screening method, after induction with 5-thio-glucose. Citrate was used as sole carbon source. The production of S65TGFP was induced in A. niger AB6.4ΔpepE[pAN52-10S65TGFPn/s]Ac5 using different initial 5-thio-glucose concentrations.

### EXPERIMENTAL PART

The effect of a non-metabolizable analog (5-thio-glucose) of D-glucose on the glaA controlled S65TGFP synthesis was studied by means of the above specified screening method.
A. niger AB6.4ΔpepE[pAN52-10S65TGFPn/s]Ac5 was grown on citrate in the presence of 5-thio-glucose. This non-metabolizable analog of D-glucose induced the production of S65TGFP under the control of the glaA promoter. This was detected via intensive fluorescence, measured by means of the above specified screening method (**Figure 2**). The concentration of this non-metabolizable inducer had a significant influence on the glaA controlled gene expression. The analog of D-glucose caused a carbon repression effect.

At a 5-thio-glucose concentration of about 0.25 mmol·L⁻¹ a maximum of the S65TGFP fluorescence intensity (RI ≅ 120%) was reached. A content of 5-thio-glucose above a concentration of 0.25 mmol·L⁻¹ caused a decrease of the S65TGFP fluorescence intensity. At a 5-thio-glucose concentration of about 33 mmol·L⁻¹ the relative fluorescence intensity (RI ≅ 17%) was only a seventh of the maximal S65TGFP fluorescence. An even higher concentration of this glucose analog (c_{Thio} ≅ 66 mmol·L⁻¹ ) resulted in an almost complete repression of the *glaA* controlled gene expression. The maximum of S65TGFP production in respect of the concentration of a non-metabolizable D-glucose analog can be explained by two conflicting regulatory mechanisms. In nature such regulatory mechanisms can regulate the glucoamylase expression in dependence of glucose concentration. Our findings fit well into a general scheme proposed for the gene regulation of polymer degrading enzymes (Hondmann and Visser 1994). According to this scheme polymer degrading enzymes are secreted at a low constitutive levels. When the respective polymer is present in the medium, this leads to the production of so called 'signal molecules' (Hondmann and Visser 1994). These 'signal molecules' act as inducers for the synthesis of a subset of enzymes, required for a rapid breakdown of the polymer. The induction of the glaA controlled S65TGFP expression by the starch hydrolyzates (maltose and glucose respectively) fits well into this concept. At high concentrations of the analog of an easily metabolized carbon source (glucose) glucoamylase synthesis was repressed. The mechanism of this glucose carbon repression is unknown. Our results indicated, that the molecule of the D-glucose analog is involved in this regulation mechanism, since 5-thio-glucose can not be metabolized. Our results showed, that glucose repression of the glucoamylase production is regulated at the level of transcription, since not only production of glucoamylase (Fang and Li 1996; Nandakumar et al. 1999) but also glaA controlled expression of the heterologous S65TGFP gene was repressed by D-glucose analogs. 5-Thio-glucose is an interesting inducer of glaA controlled protein production in bioreactor cultivations. Since only low 5-thio-glucose concentration was needed for maximal S65TGFP production (0.25 mmol·L-1), the impact of the considerable price for this non-metabolizable inducer on production costs is limited. The application of this non-metabolizable inducer in bioprocesses could lead to a new approach for optimization of induced protein production in A. niger. Induction could be carried out, using an optimal inducer concentration and growth conditions, optimized independently of the induction strategy. Whereas, when using a nutrient-inducer (e.g. maltose), this strategy (e.g. inducer puls and feeding of inducer, respectively) has a significant influence on the growth rate. The use of a non-metabolizable inducer could make the optimization of induction efficiency less complex and would therefore result in a faster process development. Using 5-thio-glucose, even a low inducer concentration, optimal for a high protein production rate, can easily be kept constant. In this way the productivity of protein production could be increased significantly.

### CONCLUSIONS

We developed a rapid screening method for the investigation of protein production under the control of the glaA promoter, using S65TGFP as fluorescent reporter protein. The screening method was successfully applied to study the influence of a non-metabolizable analog of D-glucose (5-thio-glucose). 5-Thio-glucose was detected to be a inducer of the glucoamylase gene expression system. The maximum of gene expression in respect of the concentration of this inducer was at a concentration of 0.25 mmol·L-1. For higher inducer concentrations a carbon repression effect was detected.

### NOMENCLATURE

| | |
|---|---|
| cThio | concentration of 5-thio-glucose |
| GFP | green fluorescent protein |
| RI | relative luminescence intensity |
| S65TGFP | mutant form of "green fluorescent protein" |

### MATERIALS AND METHODS

### Non-metabolizable analog of D-glucose

5-Thio-glucose is the nearest analog of D-glucose in both chemical and physical properties (Chen and Whistler 1975). It differs from D-glucose only by having a sulfur atom in place of the oxygen atom in the pyranose ring.

### Recombinant strain

The construction of the recombinant A. niger (AB6.4ΔpepE[pAN52-10S65TGFPn/s]Ac5) expressing S65TGFP under the control of the glucoamylase promoter (glaA promoter) was described previously (Siedenberg et al. 1999).

### Medium and culture conditions

Spores were obtained from 3.8% (w·v-1) potato-dextrose-agar plates, suspended in 0.9% (w·v-1) NaCl solution, and used for inoculation. A defined growth medium (modified Vogel's medium) without addition of vitamins and with ammonia as sole nitrogen source was used (Vogel 1956). The composition of the basic defined batch medium was as follows: Na3·Citrate·2H2O, 2.85 g·L-1; KH2PO4, 5 g·L-1; (NH4)2SO4, 7 g·L-1; CaCl2·2H2O, 0.1 g·L-1; MgSO4·7H2O, 0.2 g·L-1; and trace elements (citric acid·H2O, 5 mg·L-1; ZnSO4·7H2O, 5 mg·L-1; Fe(NH4)2(SO4)2·6H2O, 1 mg·L-1; CuSO4, 0.16 mg·L-1; MnSO4·H2O, 0.037 mg·L-1; H3BO3, 0.05 mg·L-1; and Na2MoO4·2H2O, 0.05 mg·L-1). The trace element solution was stored as 10,000-fold concentrated sterile-filtered stock solution. The salt solution (Na3·Citrate·2H2O, KH2PO4, NH4NO3, and CaCl2·2H2O) was stored as 50-fold concentrated sterile-filtered stock solution.

### Rapid screening studies on the performance of the glucoamylase expression system of Aspergillus niger

A method for reproducibly cultivating *A. niger* AB6.4Δ*pepE*[pAN52-10S65TGFPn/s]Ac5 as confluent mycelium layer on the bottom of 24well tissue culture plates (Falcon, Becton Dickinson, Lincoln Park, USA) was developed. A stock culture of spores, frozen at -70° C, was used for inoculation (4·10⁴ spores·mL⁻¹) to a final volume of 1.6 mL per well. The sole sugar component of the synthetic medium (above specified modified Vogel's medium) was non-inducing xylose (10 g·L⁻¹). A confluent mycelium layer and mycelium free supernatant was reached at the following cultivation conditions: 100 rpm, 96h, 30°C . The relative fluorescence intensity of this layer was always low (RFI < 10%). For the subsequent change of medium, modified Vogel's media (1.5 mL) were used (**Figure 1**). As positive control and internal standard maltose induction was carried out in 5 wells of each plate. In another well, cultivation on xylose was used as negative control. Each plate also contained three additional media (modified Vogel's medium), differing solely in the concentration of 5-thio-glucose. After incubation (100 rpm, 24h, 25°C) the *in vivo* GFP fluorescence intensities were measured automatically (excitation: 485 to 505 nm; emission: 530 to 560 nm; sensitivity: 90; average of 30 measurements), using a multi-well plate reader (Type CytoFluor™ 4000, PerSeptive Biosystems, Farmingham, USA).

### REFERENCES

Chen, M and Whistler, RL. 1975. Action of 5-thio-D-glucose and its 1-phosphate with hexokinase and phosphoglucomutase. Arch. Biochem. Biophys 169:392-396.

Fang, S and Li, M. 1996. Regulation of raw starch-digesting glucoamylase biosynthesis by *Aspergillus niger* S1. Ind. Microbiol. 26:1-6.

Hata Y, Kitamoto K, gomi K, Kumagai C, Tamura G. Functional elements of the promoter region of the Asperigillus oryzae glaA gene encoding glucoamylase.
Curr Genet 1992 Aug; 22 (2):85-91

Hondmann, D, Visser, J. Carbon metabolism; In: Martinelli, S. D. and Kinghorn, J. R. editors. Amsterdam, Lausanne, New York, Oxford, Shannon, Tokio: Elsevier, 61-139.

James JA, Berger JL, Lee BH. Purification of glucoamylase from Lactobacillus amylovorus ATCC 33621. Curr Microbiol 1997 Mar; 34(3):186-91

Lee JW, Kang DO, Kim BY, Oh WK, Mheen TI, Pyun YR, Ahn JS.Mutagenesis of the glucoamylase signal peptide of Saccharomyces diastaticus and functional analysis in Saccharomyces cerevisiae. FEMS Microbiol Lett 2000 Dec 1; 193(1):7-11

Nandakumar, MP, Thakur, MS, Raghavarao, KSMS, Ghildyal, NP. 1999. Studies on catabolite repression in solid-state fermentation for biosynthesis of fungal amylases. Lett. Appl. Microbiol. 29:380-384.

Siedenberg, D, Mestric, S, Ganzlin, M, Schmidt, M, Punt, PJ, van den Hondel, CAMJJ, Rinas, U. 1999. *GlaA* promoter controlled production of a mutant green fluorescent protein (S65T) by recombinant *Aspergillus niger* during growth on defined medium in batch and fed-batch cultures. Biotechnol. Prog. 15:43-50.

Smith TL, Gasekell J, Berka RM, Yang M, Henner DJ, Cullen D. The promoter of the glucoamylase-encoding gene of Asperigillus niger functions in Ustilago maydis. Gene 1990 Apr 16; 88(2):259-62

Sturtevant J, Dixon F, Wadsworth E, Latge JP, Zhao XJ, Calderone R. Identification and cloning of GCA1, a gene that encodes a cell surface glucoamylase from Candida albicans. Med Mycol 1999 Oct;37(5):357-66

Vogel, HJ. 1956. A convenient growth medium for *Neurospora* (Medium N). Microbial Genet. Bull. 243:112-119.

Wiebe MG, Robson GD, Shuster J, Tinci AP. Evolution of a recombinant (glucoamylase-producing) strain of Fursrium venenatum A3/5 in chemostat culture. Biotechnol Bioeng 2001 Apr 20;73(2):146-56

## Claims

1. Process for the production of a protein by means of a microorganism, wherein
(i) a microorganism is used which is able to express a homologue or heterologue protein under the control of a promoter which is a glucoamylase-synthesis controlling promoter,
(ii) the expression is carried out in the presence of a non-metabolizable expression inductor and
(iii) the expressed protein is obtained.

2. A process according to claim 1, wherein
(i) a promoter of or from a strain of Aspergillus, Saccharomyces, Candida or Lactobacillus or
(ii) a promoter is used which is encoded by a DNA, the complementary strand of which is hybridizable with the DNA encoding the promoter according to (i) under stringent conditions and which has a homology with said promoter according to (i) of at least 70 %, 80 %, 90 % or 95 %.

3. A process according to claim 2, wherein as promoter according to (i) a promoter of or from a strain of Aspergillus niger or Aspergillus oryzae is used.

4. A process according to claim 3, wherein the glaA promoter of or from A. niger is used.

5. A process according to any of the preceding claims, wherein the microorganism which is used for the expression of the protein is selected from the group of Aspergillus strains, Saccharomyces strains, Candida strains and Lactobacillus strains.

6. A process according to claim 5, wherein the Aspergillus strain is selected from the group of A. niger and A. oryzae strains.

7. A process according to any of the preceding claims, wherein an A. niger strain according to EURO FUNG BIO4CT 960 535 is used.

8. Process according to any of the preceding strains, wherein the promoter is that of or from a strain which belongs to the same genus, species or strain of the microorganism which is used for the expression of the protein.

9. A process according to any of claims 1 to 8, wherein as non-metabolizable expression inductor an analogue of a starch hydrolysation product is used
(i) which has been subjected to a test for induction of the intended protein expression, found as inducing expression and as being not subject to a metabolization, and
(ii) which has been selected.

10. A process of any of the preceding claims, wherein as non-metabolizable expression inductor an analogue of a starch hydrolysation product is used, wherein at least one oxygen atom has been exchanged by a sulfur atom.

11. A process according to any of the preceding claims, wherein as analogue of a starch hydrolysation product glucose or maltose is used wherein at least one oxygen atom has been exchanged by a sulfur atom.

12. A process according to claim 11, wherein as mon-metabolizable expression inductor 5-thio-glycose is used.
